# EUROPEAN PATENT SPECIFICATION

(11) **EP 3 586 966 B1**
(45) Date of publication and mention of the grant of the patent: **14.04.2021**
(21) Application number: 19181109.0
(22) Date of filing: 19.06.2019
(51) Int. Cl.: B01L 3/00, A61M 39/10, C12M 1/00, F16L 23/04

(54) **VESSEL CLOSURES AND METHODS FOR USING AND MANUFACTURING SAME**
BEHÄLTERVERSCHLÜSSE SOWIE VERFAHREN ZUR VERWENDUNG UND HERSTELLUNG DAVON
FERMETURES DE RÉCIPIENT ET LEURS PROCÉDÉS D'UTILISATION ET DE FABRICATION

(30) Priority: 22.06.2018 US 201816015256
(43) Date of publication of application: 01.01.2020
(73) Proprietor: Sartorius Stedim North America Inc., Bohemia, NY 11716 (US)
(72) Inventor: ZUMBRUM, Michael A., New Oxford, Pennsylvania 17350 (US)
(74) Representative: Vigand, Philippe

(56) References cited:
- WO-A1-2017/082895
- US-A1- 2005 132 821
- US-A1- 2009 119 886
- US-A1- 2015 174 514
- US-A1- 2016 311 674

## Description

### PRIORITY

The present application claims priority as a continuation-in-part to pending Application No. 14/128,259, filed March 19, 2014, which is a National Stage Entry of PCT/US2011/041462, filed June 22, 2011.

### TECHNICAL FIELD

This disclosure relates generally to vessel closures and more specifically to vessel closures having inserts, such as tubing, extending through the vessel closure.

### BACKGROUND

During certain manufacturing processes, vessels containing various fluids are used. Often it is necessary to transfer fluid into or out of a vessel during the process and do so in a substantially aseptic manner without breaching the closed nature of the system. In particular, the need to transfer fluid often arises in the manufacturing and processing of pharmaceuticals, biopharmaceuticals, or other biotechnology applications where processes are conducted in vessels of varying shapes and sizes. The need for fluid transfer into and out of a vessel arises in other applications and industries as well, including but not limited to the production of food, cosmetics, paint, chemicals, including hazardous chemicals, and the transfer and handling of semiconductor fluids.

Regardless of the industry, during transfers or sampling, the fluid in a vessel must remain substantially free of contaminants. In addition, when making such transfers, it is desirable to keep the environment surrounding a vessel free from contamination by the contents of the vessel or a sample taken therefrom. It is often the case that, throughout the manufacturing process, there is a need to maintain fluid communication with the interior of the vessel by way of, for example, tubing extending through a vessel closure from the exterior of the vessel into the interior of the vessel. To accomplish a substantially aseptic transfer, it is desirable to control the environment through which the fluid flows, for example, the pathway from a vessel to a sample container should be substantially aseptic along the entire pathway. Furthermore, it is desirable that the vessel closure be safe for use, reliable, and of low-cost construction.

It is also desirable to transfer fluid using a vessel closure which is pre-sterilized and disposable. A pre-sterilized, disposable vessel closure avoids the need for an operator to sterilize the vessel closure for use. Further, such sterilization can damage vessel closures and render them useless before their first use.

Known vessel closures utilize either thermoplastic or elastomeric tubing to create fluid communication with the interior of a vessel. Known vessel closures use, for example, all elastomeric tubing, such as silicone tubing, or utilize all thermoplastic tubing, such as C-Flex® tubing. Vessel closures incorporating elastomeric tubing are often manufactured using insert injection molding at temperatures between 350°F (177°C) to 400° (204°C). These temperatures will melt or damage thermoplastic tubing. Further, vessel closures manufactured with silicone seals have not heretofore included thermoplastic tubing due to the lack of adhesion of silicone directly onto thermoplastic tubing.

Thus, what is needed is a vessel closure with a seal capable of adhering to silicone tubing or other inserts. What is also needed is a vessel closure with a seal with both elastomeric and thermoplastic tubing within the same vessel closure wherein the tubing creates a substantially aseptic fluid pathway into and out of a vessel. What is further needed are manufacturing techniques for manufacturing a low cost, disposable vessel closure with both elastomeric and thermoplastic tubing wherein the tubing creates a substantially aseptic fluid pathway into and out of a vessel. What is also needed is a method of using the aforementioned vessel closures to circulate fluid into and out of a vessel while also having the option of controlling the temperature of the fluid.

### SUMMARY

Briefly described, a vessel closure comprising a body, one or more apertures extending axially through the body, one or more inserts extending axially through one or more apertures, and a cast seal disposed within the body and surrounding each insert. In one embodiment, the vessel closure may have one or more inserts comprising silicone tubing. In one embodiment, the cast seal is pourable, self-leveling silicone, such as room temperature vulcanizing ("RTV") silicone.

According to the invention, a gasket is disposed between a pair of vessel closures, which are held together by a clamp, to form a fluid transfer hub. The fluid transfer hub can create a manifold where fluid from a source is distributed to a plurality of vessels A fluid transfer hub according to the invention is defined in claim 1.

In another embodiment, briefly described, a tubing assembly in a vessel closure comprising one or more thermoplastic tubes extending axially through one or more apertures extending through the vessel closure; an anchor adhesively attached to a portion of each thermoplastic tube, the anchor capable of bonding with a casting agent; and a casting agent securing each anchor and thermoplastic tube through each aperture. The thermoplastic tubes may be C-Flex® tubing.

In another embodiment, briefly described, a tubing assembly in a vessel closure comprising flexible tubing extending through an aperture in the closure; a deformable sleeve adhesively attached to and surrounding at least a portion of the flexible tubing; the sleeve being formed of a material having plasticity such that pressure applied to the sleeve causes the sleeve to deform about and seal the flexible tubing and upon continued application of pressure to the sleeve, the sleeve and flexible tubing are cut and the sleeve retains a deformed shape substantially sealing the tubing; and a cast seal surrounding the sleeve.

In a further embodiment, briefly described, a vessel closure comprising a body, at least two apertures through the body, and flexible tubing passing through the at least two apertures wherein the ends of the tubing are both directed into the interior of the vessel thereby forming a loop on the exterior of the vessel.

Also, briefly described, a method of manufacturing a vessel closure comprising a body, one or more apertures extending axially through the body, and an insert extending axially through one or more apertures. The method comprising creating one or more apertures in a body; inserting an insert axially through one or more apertures; casting a casting agent into the body; and curing the casting agent to form a seal around and bond to the one or more inserts A method for providing a fluid transfer hub according to the invention is defined in claim 13.

In another embodiment, briefly described, a method of manufacturing a vessel closure, the vessel closure comprising one or more apertures through a body with tubing extending through the one or more apertures; the method comprising creating one or more apertures in a body; inserting silicone tubing through at least one aperture; inserting thermoplastic tubing through at least one other aperture wherein at least a portion of the thermoplastic tubing is surrounded by a sleeve adhesively attached to the tubing, wherein the outer surface of the sleeve is capable of bonding with a casting agent; casting a casting agent into the body; and curing the casting agent to form a seal around and bond to the silicone tubing and to the at least one sleeve surrounding the thermoplastic tubing. In one embodiment, the thermoplastic tubing may be C-Flex® tubing.

Also, briefly described, a method of circulating a fluid into and out of a vessel comprising sealing a vessel with a closure, the closure comprising a body, at least two apertures through the body, and flexible tubing routed through the at least two apertures so the ends of the tubing are both directed into the interior of the vessel thereby forming a loop on the exterior of the vessel; attaching circulating means to the loop on the exterior of the vessel and using circulating means to circulate fluid out of and into the vessel through the flexible tubing. In one embodiment, the fluid is circulated using a peristaltic pump.

Thus, vessel closures, methods of manufacturing the same, and methods of utilizing the same are disclosed that possess distinct attributes and represent distinct improvements over the prior art. These and other aspects, features, and advantages of the vessel closures of this disclosure will be better understood and appreciated upon review of the detailed description set forth below when taken in conjunction with the accompanying drawing figures, described briefly below. According to common practice, the various features of the drawings may not be drawn to scale. Dimensions and relative sizes of various features and elements in the drawings may be shown enlarged or reduced to illustrate more clearly the embodiments of the invention.

### BRIEF DESCRIPTION OF THE DRAWINGS

Fig. 1 is a perspective view of a vessel closure, cast seal, and three tubes.
Fig. 2 is a bottom view of a vessel closure, cast seal, and three tubes.
Fig. 3 is a cross section of a vessel closure, cast seal, and three tubes.
Fig. 4 is a cross section of a vessel closure, cast seal, three tubes, and three different anchors, illustrating a variety of anchors that may be used.
Fig. 5 is a side view of a vessel closure, cast seal, and two tubes, illustrating the seal pulled away from the interior of the vessel closure.
Fig. 6 is a cross-section of a vessel closure, cast seal, and two tubes, illustrating the vessel closure in a relatively smaller vessel closure than shown in Fig. 1.
Fig. 7 is a cross-section of a vessel closure, cast seal, two tubes, illustrated in yet a different relatively smaller vessel closure.
Fig. 8 is a perspective view of a vessel closure, cast seal, and two tubes.
Fig. 9 is a cross-section of a vessel closure, cast seal, two tubes, and an aperture in the vessel closure sealed only by the cast seal, illustrating a pierceable section of the seal.
Fig. 10 is a cross-section of a vessel closure, cast seal, two tubes, and a puncture site, illustrating an embodiment with an anchor attached to one tube, a tube with no anchor, and a specialized puncture site over one aperture in the vessel closure.
Fig. 11 is a cross-section of a vessel closure depicting a temperature probe, tube, and anchor.
Fig. 12 is a side view of a tube and anchor.
Fig. 13 is a perspective view of a vessel closure, temperature probe, tube, and anchor before the tube and anchor are connected to the vessel closure and before a seal is cast around the anchor, tube, and probe.
Fig. 14 is a cross-section of a vessel closure, two fittings, and a tube wherein the fittings shown are a male and female luer.
Fig. 15 is a perspective view of a tri-clamp closure, three tubes, and a cast seal.
Fig. 16 is a cross-section of a tri-clamp closure, three tubes, and a cast seal.
Fig. 17 is a cross-section of a closure, a tube, and fitting wherein the fitting shown is a quick connect fitting.
Fig. 18 is a side view of a closure and two fittings wherein the fittings are a barbed fitting and a quick connect fitting.
Fig. 19 is a cross-section of a vessel closure and tubing, illustrating the tubing routed through the closure to form a loop on the exterior of the closure.
Fig. 20 is a cross-section of a vessel, vessel closure, seal, and silicone tube, illustrating the vessel closure sealed to the vessel.
Fig. 21 is a perspective view of a fluid transfer hub formed from a pair of vessel closures.
Fig. 22 is an exploded view of the fluid transfer hub.
Fig. 23 is a cross-section of the fluid transfer hub according to a first embodiment.
Fig. 24 is a cross-section of the fluid transfer hub according to a second embodiment.
Fig. 25 is a cross-section of a cast form suitable for manufacturing a vessel closure of the fluid transfer hub of Fig. 24.

### DETAILED DESCRIPTION OF EXEMPLARY EMBODIMENTS

Certain exemplary embodiments of the present invention are described below and illustrated in the accompanying figures. The embodiments described are only for purposes of illustrating the present invention and should not be interpreted as limiting the scope of the invention, which of course, is limited only by the claims below. Other embodiments of the invention, and certain modifications and improvements of the described embodiments, will occur to those skilled in the art, and all such alternate embodiments, modifications, and improvements are within the scope of the present invention.

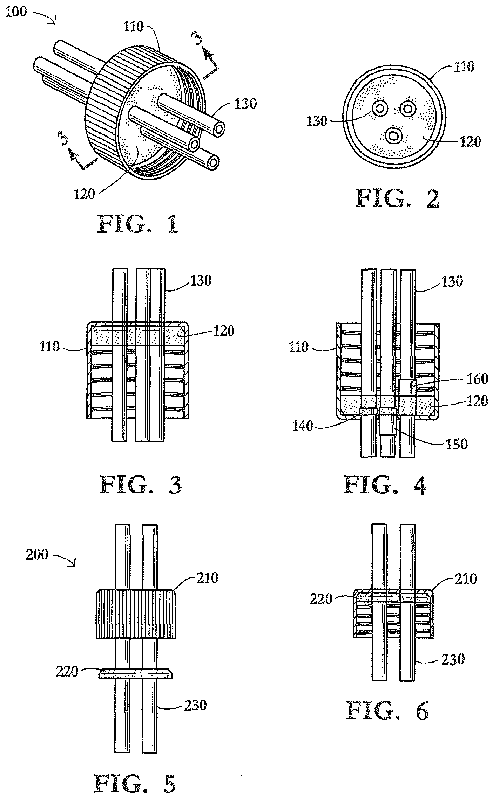

Referring now in more detail to the drawing figures, wherein like reference numerals indicate like parts throughout the several views, Fig. 1 depicts a first exemplary vessel closure, in this case a vessel closure with multiple tubes extending through the vessel closure thereby creating fluid communication between the interior and exterior of a vessel onto which the vessel closure may be placed. It should be understood that the vessel closure is not limited to use with any particular fluids but, depending on the size and composition of the inserts, may be used with fluids with particulates or having a high viscosity or with fluids having no or very little particulate content or low viscosity.

The vessel closure **100** illustrated in Fig. 1 generally depicts a body, inserts, and a seal shown assembled together to form a single vessel closure. The vessel closure **100** has a body **110,** discussed in more detail below, one or more apertures extending axially through the body, inserts **130** extending axially through each aperture, and a cast seal **120** disposed within the body and surrounding each insert. The vessel closure is not limited to this number of inserts, but may incorporate more or fewer inserts. The vessel closure is suitable for use with vessels that include without limitation: containers, beakers, bottles, canisters, flasks, bags, receptacles, tanks, vats, vials, tubes, and the like that are generally used to contain fluids, slurries, and other similar substances.

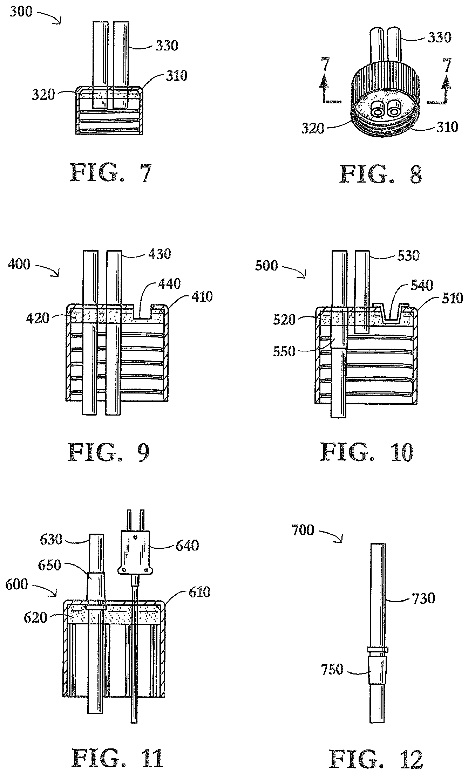

In the vessel closure illustrated in Fig. 1, the body is a cap. Suitable caps for the vessel closure include those commonly used in the field of pharmaceutical, biopharmaceutical, and biotechnology processing. Such caps include: a 38-430 cap with an outer diameter at the open end of approximately 42 mm and being approximately 29 mm tall (as depicted in Figs. 1, 4, 9, 10, and 17-19); a centrifuge cap having an outer diameter at the open end of approximately 34 mm and being approximately 13 mm tall (such as depicted in Figs. 5 and 6); a 20-415 cap with an outer diameter at the open end of approximately 24 mm and being approximately 14.6 mm tall (such as depicted in Figs. 7 and 8); and a GL-45 cap having an outer diameter at the open end of approximately 53.7 mm and being approximately 25.5 mm tall. The invention, however, is not limited to a cap of any particular dimensions. Bodies of vessel closures may be made from thermoplastics such as polyolefins, polypropylene, polyethylene, polysulfone, polyester, polycarbonate, and glass filled thermoplastics. The invention, however, is not limited to a cap made from any particular material(s). The bodies may also be made from thermosets such as epoxies, pheonolics, and novolacs. The body may also be a hygienic or sanitary clamp having dimensions disclosed in ASME BPE table DT-5-2 ("Hygienic Clamp Ferrule Standard Dimensions and Tolerances") (2009. The body is not limited to caps or hygienic clamps but may constitute any suitable closure that seals the interior of a vessel from the exterior environment.

Fig. 1 further depicts three inserts **130** extending axially through the body. The inserts extend through apertures in the body. The number of apertures in the body may correspond with the number of inserts to be placed in the body.

Also depicted in Fig. 1 is a cast seal **120.** In a preferred embodiment the cast seal is constructed from a self-leveling, pourable silicone such as room temperature vulcanizing ("RTV") silicone. The RTV silicone may be a two-component system (base plus curative) ranging in hardness from relatively soft to a medium hardness, such as from approximately 9 Shore A to approximately 56 Shore A. Suitable RTV silicones include Wacker® Elastocil® RT 622, a pourable, addition-cured two-component silicone rubber that vulcanizes at room temperature; and Blue Star Silicones Rhodorsil® RTV 1556, a two-component, high strength, addition-cured, room temperature or heat vulcanized silicone rubber compound. Both the Wacker® Elastocil® RT 622 and the Bluestar Silicones Rhodorsil® RTV 1556 have a viscosity of approximately 12,000 cP (mPa.s). The aforementioned silicones and their equivalents offer low viscosity, high tear cut resistance, high temperature and chemical resistance, excellent flexibility, low shrinkage, and the ability to cure a cast silicone seal at temperatures as low as approximately 24° C (75° F). In another embodiment, the casting agent is a perfluoropolyether liquid. A preferred perfluoropolyether liquid is Sifel 2167, available from Shin-Etsu Chemical Co., Ltd.

In an embodiment, the cast seal is disposed within the body of the vessel closure so that when the body is attached to a vessel opening, such as a bottle opening, the cast seal creates a seal between the interior of the vessel and the exterior environment. Preferably, the seal formed by the vessel closure between the interior of the vessel and the exterior environment is substantially aseptic. As best shown in Figs. 1 and 3, the cast seal **120** surrounds the inserts **130,** thereby creating a unified seal and insert subassembly. In an embodiment, the seal between the cast seal **120** and insert **130** is substantially aseptic.

In one embodiment, the inserts are silicone tubing. For clarity, only a portion of the silicone tubing is shown in Fig. 1. The tubing may be of any length suitable and necessary for the desired process. In an embodiment, at least a portion of the silicone tubing is treated with a primer where the cast silicone surrounds the tubing. Suitable primers are SS-4155 available from Momentive™, Med-162 available from NuSil Technology, and Rodorsil® V-O6C available from Bluestar Silicones.

Referring now to Fig. 2, a bottom-view of a vessel closure shows the body 110, the inserts **130,** and the cast seal **120** partially filling the body of the vessel closure and surrounding the exterior of the inserts extending through the apertures in the body. As shown in Fig. 3, which is a cross-section of the vessel closure, the body **110** contains the cast seal **120** and the inserts extend axially through the body **110** and the cast seal **120.**

Turning now to Fig. 4, a vessel closure is shown with a variety of anchors **140, 150,** and **160.** For clarity, the selection and depiction of various anchors is for illustrative purposes only. The vessel closures may have any variety of different anchors, have all the same anchors, or no anchors at all. Specifically, Fig. 4 depicts three different anchors **140, 150,** and **160,** a body **110,** a cast seal **120,** and three inserts **130,** in this case, tubing. Anchor **140** is a retaining nut affixed in place around the silicone tube insert **130.** The retaining nut prevents the insert from being pulled out of a vessel. Anchor **150** has a retaining nut portion and a cone-shaped portion. (Anchor **150** is also shown as anchor **750** in Fig. 12.) The retaining nut portion is located on the interior of the body, while the cone-shaped section is on the exterior of the body. Anchor **150** prevents the insert from being pulled out of a vessel or being pulled into a vessel when, for example, a vacuum is placed on the interior of the vessel. Anchor **160** is a sleeve, also referred to herein as an anchor sleeve, that prevents the insert from being pulled out of the body. Anchor sleeve **160** also provides a surface to which the cast seal may bond. The cast seal may also bond to an anchor and the insert on which the anchor is placed. Regardless of the shape of the anchor, the cast seal **120** bonds to the anchor. The anchors may be constructed of such material that is particularly suitable for bonding with the cast seal, such as metal (aluminum, stainless steel, etc.), plastic, glass-filled plastic, ceramics, and composites. In an embodiment, the anchors are adhesively attached to the inserts. Thus, when an embodiment of a vessel closure is constructed, the anchor is adhesively attached to the insert, for example, a tube; the cast seal is bonded to the anchor, and the anchor secures the one or more tubes and cast seal to the body of the vessel closure.

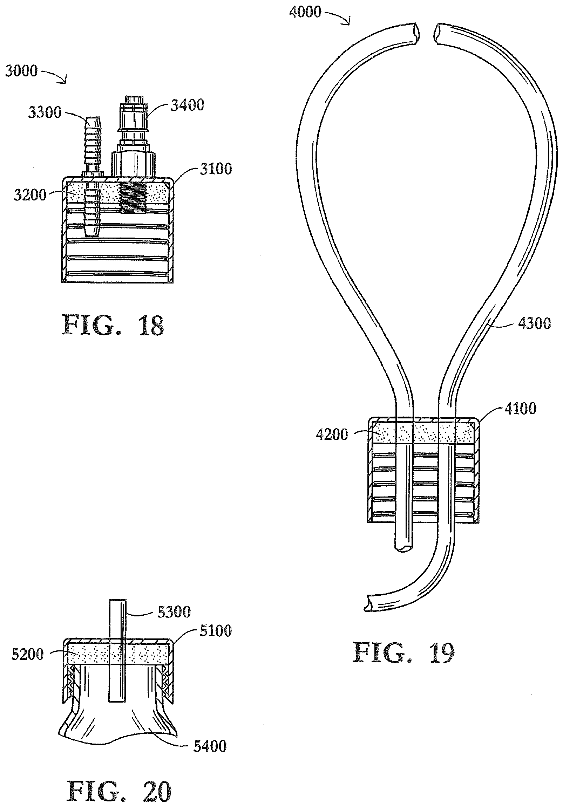

In another embodiment, the cast seal **120** is attached to the body by way of priming at least a portion of the body and adhesively attaching the cast seal to the body. In this embodiment, the cast seal will not pull away from the interior of the body. However, in other embodiments the cast seal need not necessarily be attached to the body. As best shown in Fig. 5, a vessel closure **200** may have a body **210,** apertures extending axially through the body (not shown in Fig. 5), a cast seal **220,** and two inserts **230,** in this case, silicone tubing. The body shown in Fig. 5 represents a centrifuge cap. In Fig. 5, the cast seal **220** is shown pulled away from the body **210.** Upon placement of the body of the vessel closure to a vessel, for example, placing a cap on a bottle, the cast seal **220** will be seated within the body. Fig. 20 shows such an arrangement and is discussed in detail below. Fig. 6 shows the cast insert seated within the body **210** without the presence of a vessel. As with the vessel closure shown in Fig. 1, the vessel closure in Fig. 4 shows two inserts but any number of inserts could be used.

Returning to Fig. 4, the vessel closure may have only inserts such as the insert with anchor sleeve **160.** In an embodiment, the anchor sleeve **160** adhesively attaches to the insert. In one embodiment, the insert is thermoplastic tubing, such as C-Flex® tubing. When anchor sleeves partially or completely surround each insert, the seal need not be constructed of cast silicone but may be made of any casting agent capable of bonding to the anchor sleeve. For example, in applications involving solvents, a casting agent such as liquid perfluoropolyether could be used. Regardless of which casting agent is used, the casting agent is preferably aseptically sealed to the anchor.

In an embodiment, at least a portion of the anchors are treated with a primer where the anchor sleeve contacts the casting agent. In yet another embodiment, the vessel closure may contain inserts comprising elastomeric tubing wherein at least a portion of the tubing is surrounded by an anchor sleeve and silicone tubing extends through the body and seal. In such embodiment, the silicone tubing is not surrounded, either partially or wholly by an anchor sleeve. In this embodiment, the preferred casting agent is liquid silicone.

Again returning to Fig. 4, anchor sleeve **160** may be a deformable sleeve, such as those disclosed in PCT/US08/048 8. In an embodiment, the deformable sleeve surrounds and is adhesively attached to flexible tubing. In one embodiment, the deformable sleeve is attached to the flexible tubing at a location that facilitates substantially sealing, cutting, and detaching the deformable sleeve. In such embodiments, the sleeved is formed of a material having plasticity such that pressure applied to the sleeve causes the sleeve to deform about and seal the flexible tubing and upon continued application of pressure to the sleeve, the sleeve and flexible tubing are cut and the sleeve retains a deformed shape, thereby substantially sealing the tubing. In these embodiments, the flexible tubing is thermoplastic tubing, elastomeric tubing, or a combination of thermoplastic and elastomeric tubing. Further, when a deformable sleeve is used, the casting agent may adhere to and aseptically seal with the deformable sleeve.

Turning now to Figs. 7 and 8, a vessel closure **300** is shown representing a body **310** representative of a 20-415 cap, a cast seal **320,** and two inserts **330.** The inserts may be silicone tubing.

Fig. 9 illustrates an embodiment of a vessel closure **400** having a body **410,** a cast seal **420**, inserts **430**, and a pierceable recess **440** formed in the cast seal **420**. The pierceable recess **440** is a section of the cast seal **420** in which a removable insert was set during the casting and curing of the silicone and later removed to form an area of the cast seal **420** that is thinner than the remainder of the cast seal. In one embodiment the pierceable recess **440** is thin enough for a hypodermic needle to be inserted through the pierceable recess **440** thereby accessing the interior of a vessel so that fluid may be introduced into or removed from a vessel.

Fig. 10 illustrates another embodiment of a vessel closure **500** having a body **510,** a cast seal **520,** inserts **530,** and a pierceable insert **540.** The pierceable insert **540** may be constructed from silicone or other suitable material that would allow a hypodermic or other similar needle to pierce the insert and thereby introduce or remove fluid from a vessel. Also shown in in Fig. 10 is an anchor sleeve **550.** Fig. 10 illustrates an example of a vessel closure potentially having three different inserts. The tubing without the sleeve may be silicone while the tubing with the sleeve could be elastomeric tubing, such as C-Flex® tubing. Lastly, the pierceable insert **540** represents the third type of insert.

Fig. 11 depicts yet another embodiment of a vessel closure **600** having a body **610,** a cast insert **620,** inserts **630** and **640,** and an anchor **650.** Insert **630** may be, for example, silicone tubing, and the anchor **650** is constructed with a retaining nut portion in the interior of the body **610** and a conical section on the exterior of the body **610,** thereby securing the insert **630** to body **610** by way of, for example, a friction fit. Insert **640** is a temperature probe. Various inserts may be used in the vessel closures including but not limited to: tubing (including but not limited to elastomeric and thermoplastic tubing), temperature probes, pH sensors, pierceable inserts, strain relief boots, dip tubes, barbed fittings, luers, and quick connect fittings. The invention is not limited to the aforementioned inserts. Inserts of any size, function, and construction may be used.

Further, check valves may be attached to tubing extending into the interior of a vessel. Use of such check valves keeps fluids from entering a vessel until a requisite and sufficient force is applied to the fluid to open the check valve. Such force could be applied, for example, by a syringe or pump. In one embodiment, check valves such as Model #110 from Smart Products may be used.

Fig. 12 illustrates an insert **700** comprised of tubing **730** and an anchor **750.** The anchor **750** is the same anchor as depicted in Fig. 11 and illustrates a how the anchor may be friction fit into an aperture in the body **610** by pressing the conical section into and through an aperture until the anchor snaps into place.

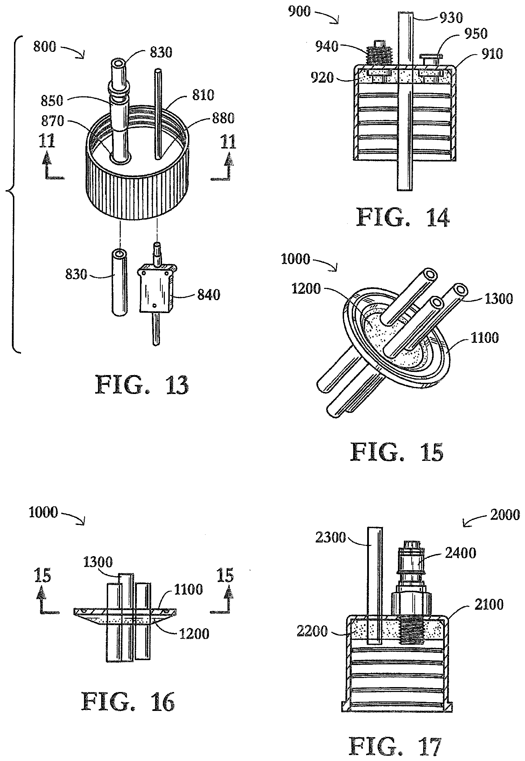

As shown best in Fig. 13, various inserts extend axially through apertures in the body during the manufacturing process before a casting agent is poured into the body of a vessel closure to form a seal. Specifically, Fig. 13 illustrates a perspective view of a vessel closure **800,** a body **810,** an insert **830,** a temperature probe **840,** an anchor sleeve **850,** and apertures **870** and **880** in the body. As shown in Fig. 13, the insert **830** and anchor sleeve **850** combination have not yet been fully inserted into aperture **870** in the body. In addition, the cast seal (not shown) has not yet been poured.

Fig. 14 illustrates a vessel closure **900,** a body **910,** a cast seal **920,** and three inserts **930, 940,** and **950.** In a preferred embodiment insert **930** is silicone tubing. Insert **940** is a male luer fitting and insert **950** is a female luer fitting.

Fig. 15 depicts a tri-clamp vessel closure **1000** comprising a body **1100,** a cast seal **1200,** and inserts **1300.** Fig. 16 shows a cross-section of the tri-clamp vessel closure **1000** comprising a body **1100,** a cast seal **1200,** and inserts **1300.** Tri-clamp closures may be made with inserts other than the tubing inserts shown in Figs. 15 and 16. Tubing is shown as merely an example of the type of insert that may be used.

Fig. 17 illustrates a vessel closure **2000,** a body **2100,** a cast seal **2200,** and two inserts **2300** and **2400.** In an embodiment insert **2300** is a silicone tube. Insert **2400** depicts a male quick connect fitting. A quick connect fitting may be further secured to the body of the vessel closure by affixing a nut to the threaded portion of the insert that extends into the interior of the body. The body **2100** represents a 38-430 cap.

Fig. 18 depicts a vessel closure **3000,** a body 3100, a cast seal 3200, and two inserts **3300** and **3400.** Insert **3300** is a barbed fitting of the type that accepts flexible tubing, such as silicone or thermoplastic tubing. Insert **3400** is a male quick connect fitting. In an embodiment, the barbed insert may have a barbed extension extending into the interior of the body as well as extending away from the exterior of the body so that tubes may be attached extending into and out of a vessel.

Fig. 19 depicts a vessel closure **4000,** a body **4100,** a cast seal **4200,** and tubing **4300.** Body **4100** has at least two apertures so that tubing **4300** passes through each aperture and the ends of the tubing are both directed into the interior of a vessel thereby forming a loop on the exterior of the vessel. The loop show in Fig. 19 is for illustrative purposes only and the loop on the inventive vessel closure may made in any length necessary to accommodate pumping or pumping and temperature control. The vessel closure illustrated in Fig. 19 may be used to circulate fluid into and out of a vessel without exposure to the environment outside of the vessel, thereby forming a closed system utilizing few components. In an embodiment, a peristaltic pump is connected to the loop of tubing outside the vessel and used to circulate the fluid. In yet another embodiment, a heat exchanger is used, instead of or in addition to a pump, to regulate the temperature of the fluid.

As best shown in Fig. 20, the cast seals disclosed herein may be sealingly compressed between the body of a vessel closure and a vessel by tightening the closure onto a vessel. Fig. 20 depicts a vessel closure body **5100** mechanically depressed to a vessel by the body being tightened to a vessel **5400** thereby compressing the cast seal **5200** against the vessel. In a preferred embodiment, the cast seal is aseptically sealed to the vessel.

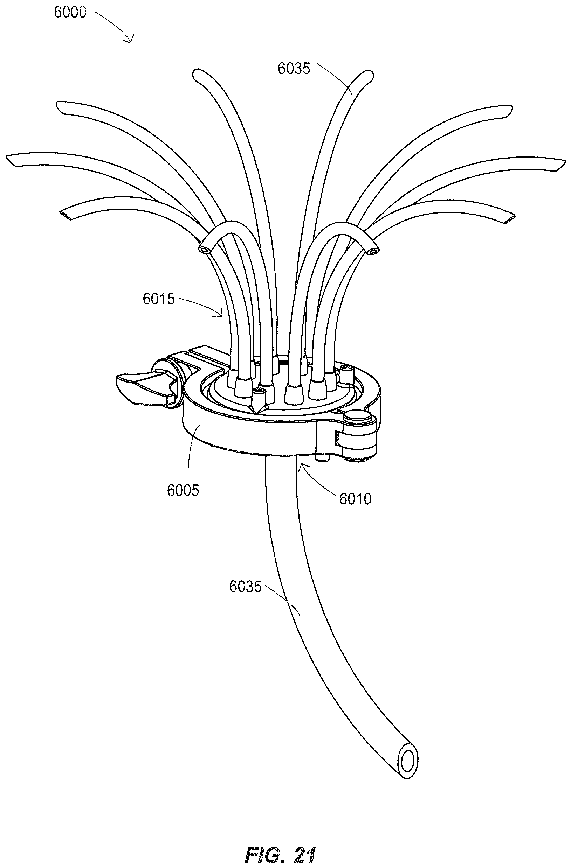

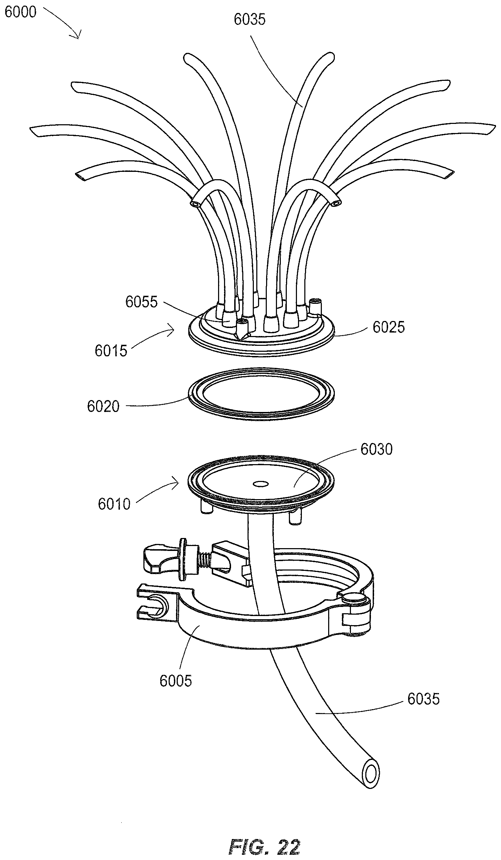

The vessel closures discussed above, particularly the tri-clamp vessel closures **1000** (Fig. 15) may be combined to create additional, alternative embodiments. Fig. 21 depicts a fluid transfer hub **6000.** Fig. 22 depicts an exploded view of the fluid transfer hub **6000** to provide a more clear illustration of the components thereof according to one embodiment. The fluid transfer hub **6000** may be comprised of a tri-clamp **6005,** a first tri-clamp vessel closure **6010,** a second tri-clamp vessel closure **6015,** and a gasket **6020.**

The tri-clamp **6005** in the illustrated embodiment is a 2-segment, single-hinge type clamp. Alternatives that are well-known to one having ordinary skill in the art include 3-segment, double-hinge clamps, and high-pressure, no-hinge type clamps. Each tri-clamp vessel closure **6010, 6015** is a tri-clamp fitting that includes a body **6025,** a cast seal **6030,** and at least one insert **6035.** The body **6025** may be substantially similar to the body **1100** of the tri-clamp vessel closure **1000** (Fig. 15). The body **6025** includes one or more apertures extending axially through the body. The body **6025** is a clamp body and can have any dimensions, but may be preferably among the standard clamp dimensions for hygienic and sanitary uses as discussed above. Further, the body **6025** may be formed from any of the suitable body materials discussed above. Similarly, the cast seal **6030** of the vessel closures **6010, 6015** of the fluid transfer hub **6000** may be formed from the same suitable materials, and formed using the same methods, as discussed above with respect to the cast seal **120** (Figs. 1-4). Further, the inserts **6035** may be silicone tubing, thermoplastic tubing, or other components consistent with the inserts discussed elsewhere in the present disclosure.

To help create a seal between the two tri-clamp vessel closures **6010, 6015,** a gasket **6020** is provided. Gaskets **6020** are available in standard sizes that corresponding with the standard dimensions of tri-clamps and their corresponding fittings. Gaskets **6020** are available in several materials, including copolymers of acrylonitrile and butadiene (BUNA-N), VITON®, fluoroelastomers as defined by ASTM D1418 (FKM), ethylene propylene diene monomer (EPDM), polytetrafluoroethylene (PTFE), silicone, and others. An open gasket **6020** is illustrated in Fig. 22, but other types of gaskets are available that may be used within the fluid transfer hub **6000.** Alternative gaskets **6020** include orifice gaskets, screen gaskets, and perforated plate gaskets that may control flow of a fluid through the fluid transfer hub **6000,** or provide a filtering function. Each of these alternative gaskets are available in several sizes, or can be customized, based upon the dimensions of the fittings, the orifice diameter through the gasket, or the pore size of the perforated plate or screen gaskets. Suitable gaskets **6020** are available from Flow Smart Inc. and others.

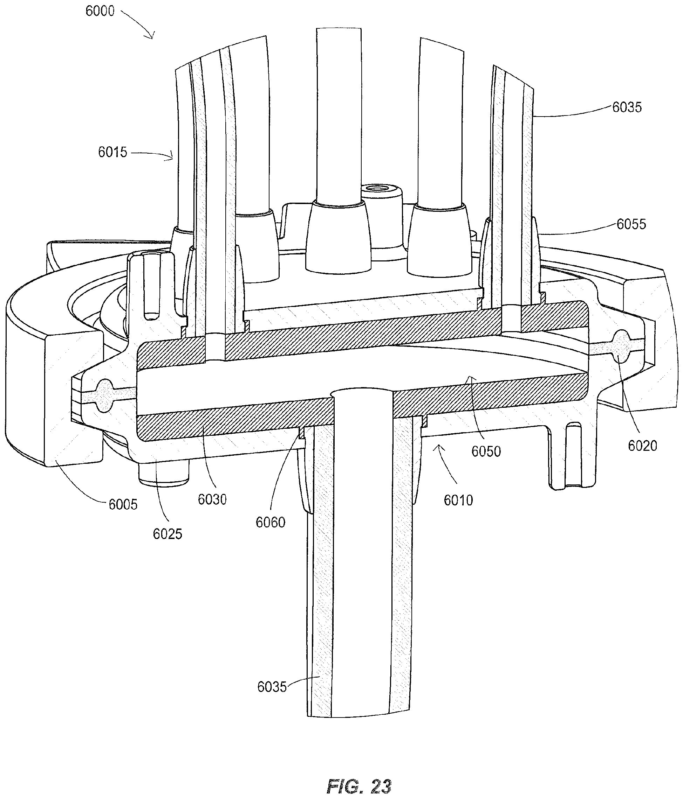

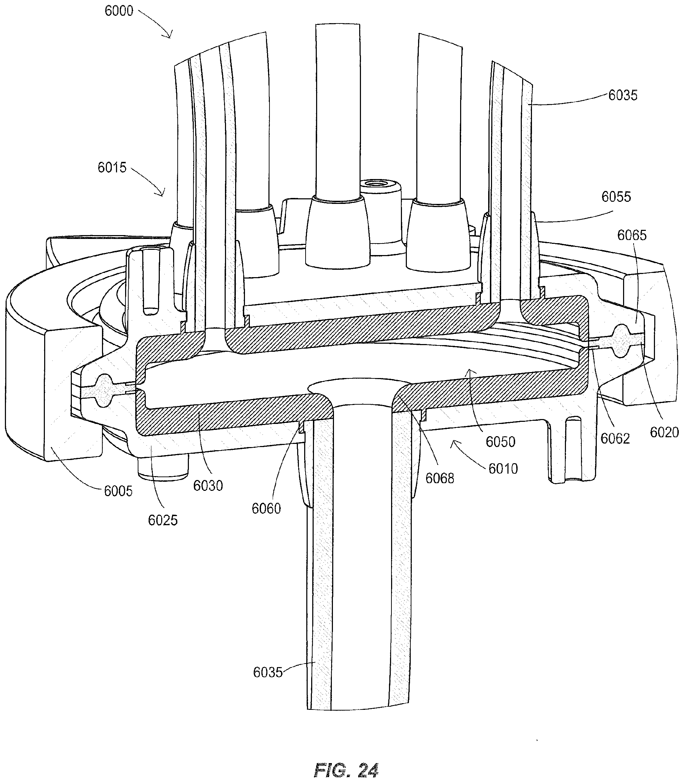

Figs. 23 and 24 illustrate alternative cross sections of a fluid transfer hub **6000.** The tri-clamp **6005,** first tri-clamp vessel closure **6010,** and second tri-clamp vessel closure **6015** are illustrated along with the gasket **6020.** The inserts **6035** of each tri-clamp vessel closure **6010, 6015** include tubing. In the illustrated embodiment, the first tri-clamp vessel closure **6010** includes only one insert **6035** providing a single inlet into a chamber **6050** from a source vessel. The second tri-clamp vessel closure **6015** includes a plurality of inserts **6035** that provide a plurality of outlets from the chamber **6050** for distributing a fluid into a plurality of receptacles. With the illustrated configuration, the fluid transfer hub **6000** may be described as a manifold. The chamber **6050** may be bisected by the screen or perforated plate of a corresponding gasket **6020,** if applicable.

The inserts **6035** pass through apertures in the bodies **6025** of each tri-clamp vessel closure **6010, 6015,** and are connected to the bodies by the cast seal **6030.** In addition, in the illustrated embodiments of Figs. 23 and 24, each insert **6035** is coupled to an optional anchor **6055** that provides an interface between the insert and the body **6025.** The tri-clamp vessel closures **6010, 6015** are illustrated with a similar anchor **6055** on each insert **6035,** however, a variety of different anchors, or no anchors at all, may also be used. Each anchor **6055** may be any of the types of anchors described above. For example, the anchor **6055** may be in the form of a retaining nut affixed in place around an insert **6035.** The retaining nut prevents the insert **6035** from being pulled out of the body **6025.** The illustrated anchor **6055** has a retaining nut portion and a cone-shaped portion. The retaining nut portion is located toward the interior of the body **6025,** while the cone-shaped section is on the exterior of the body. Further, the anchor **6055** could be a sleeve that prevents the insert **6035** from being pulled out of the body **6025,** and provides a surface to which the cast seal **6030** may bond. The anchors **6055** may be constructed of such material that is particularly suitable for bonding with the cast seal **6030,** such as metal (aluminum, stainless steel, etc.), plastic, glass-filled plastic, ceramics, and composites. In an embodiment, the anchors **6055** are adhesively attached to the inserts **6035** and combined with the vessel closures **6010, 6015** as discussed above.

Fig. 23 shows a first embodiment of the cast seal **6030.** As discussed above, the cast seal **6030** may be a self-leveling silicone that is poured into the body **6025** of each tri-clamp vessel closure **6010, 6015** after the inserts **6035** and optional anchors **6055** are in place. The cast seal **6030** may coat a bottom interior surface of the body **6025.** The cast seal **6030** may fill in a seam **6060** between the anchor **6055** and the body **6025** to increase the surface contact with the cast seal and improve the retention of the insert **6035.**

Fig. 24 shows a second embodiment of the cast seal **6030.** In Fig. 24, the cast seal **6030** includes additional material to coat both the bottom interior surface of the body **6025** as well as the interior sidewalls of each body. The cast seal **6030** is also configured to wrap onto a flange **6065** of the body **6025** to be in contact with the gasket **6020** at tip **6062.** In the illustrated embodiment, the tip **6062** of the cast seal **6030,** and therefore also the parting seam of the cast seal created by the body **6025** and the cast form (discussed below), is positioned along the top of the flange **6065** and outside of the fluid pathway through the fluid transfer hub **6000.** The illustrated positioning of the tip **6062** can help limit any potential for particles of the cast seal **6035** to enter the fluid stream. The cast seal **6030** is also represented with a rounded edge **6068** leading from the chamber **6050** into each insert **6035.** The rounded or curved configuration of the edges **6068** reduces the potential for air to become trapped in the chamber **6050** as fluid is transfers across the fluid transfer hub **6000.** The curved configuration of the edges **6068** may also improve the flow of fluid exiting the fluid transfer hub **6000** to improve the accuracy of fluid distribution from the hub to the individual receiving vessels, e.g. **5400,** Fig. 20.

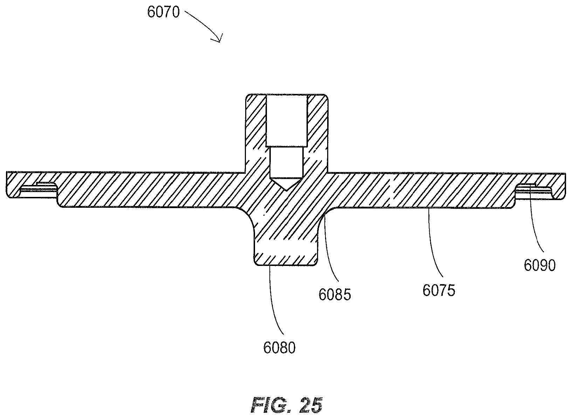

Fig. 25 shows a cast form **6070** according to one embodiment. The cast form **6070** may be a Teflon® coated aluminum tool. The cast form **6070** includes a base surface **6075** and at least one boss **6080** extending from the base surface **6075.** A curved surface **6085** may transition from the base surface **6075** to the boss **6080.** The illustrated cast form **6070** may further comprises a circumferential channel **6090** along the flange thereof. The cast form **6070** may also include small holes (not shown) drilled or otherwise provided in communication with the base surface **6075** for providing a fill port and a vent port for use in the casting process as will be understood by one having ordinary skill in the art.

The cast form **6070** of Fig. 25 is particularly suitable for manufacture of the first tri-clamp vessel closure **6010** of the embodiment shown in Fig. 24. The curved surface **6085** of the cast form **6070** is configured to create the rounded edge **6068** leading from the chamber **6050** into each insert **6035** of the first vessel closure **6010.** The quantity and arrangement of the at least one boss **6080** on the base surface **6075** is configured to correspond with the quantity and arrangement of apertures in a corresponding body **6025** of a corresponding tri-clamp vessel closure. Further, the circumferential channel **6090** of the cast form **6070** of Fig. 25 provides for the tip **6062** of the cast seal **6030** that wraps onto the flange **6065** of the first tri-clamp vessel closure **6010** of Fig. 24.

The vessel closures are manufactured by creating one or more apertures in a body. In an embodiment, the apertures may be made using a punch press. However, the apertures may be made using a drill, mill, laser, or any combination thereof. In another embodiment, the apertures are molded when the bodies are molded. Inserts are then inserted axially through the one or more apertures in the body. A casting agent is then cast into the interior of the body. The casting agent is then cured to form a seal around and bond to the one or more inserts. Preferred casting agents include liquid silicone and liquid perfluoropolyether.

In an alternative embodiment, a vessel closure comprising one or more apertures through a body with tubing extending through the one more apertures may be manufactured by first creating a body with one or more apertures. The apertures in the body may be made by the methods mentioned above. Silicone tubing is then inserted through at least one aperture. Thermoplastic tubing is then inserted through at least one other aperture so that the vessel closure has both silicone and thermoplastic tubing. A portion of the thermoplastic tubing is surrounded by a sleeve adhesively attached to the thermoplastic tubing. The sleeve is constructed of material capable of bonding with a casting agent. A casting agent is then cast into the body. In embodiments where the surface of the cast seal is contoured, such as the rise to the tip **6062** or the rounded edge **6068** illustrated in Fig. 24, the step of casting the casting agent into the body may be preceded by obtaining a suitable cast form and engaging the cast form with the body to form a cavity for the casting agent between the body and the cast form. The casting agent is cured to form a seal around and bond to the at least one sleeve surrounding at least a portion of the thermoplastic tubing and to the silicone tubing. In an embodiment, the thermoplastic tubing is C-Flex® tubing. Preferred casting agents include liquid silicone and liquid perfluoropolyether. After curing, the cast form, if present, would be separated from the body.

In the aforementioned methods for manufacturing vessel closures, the liquid silicone may be cast at temperatures much lower than required for insert and/or injection molding. Accordingly, components may be incorporated into the vessel closures that could not be incorporated into vessel closures manufactured using insert and/or injection molding. Such inserts include, but are not limited to, thermoplastic inserts such as C-Flex® tubing. Accordingly, the vessel closures may combine elastomeric and thermoplastic tubing within the same vessel closure. The vessel closures also allow for a variety of different inserts to be included in a single vessel closure, such as a variety of fittings, silicone tubing, C-Flex® tubing, temperature gauges, pH sensors, and others. The vessel closures are low cost and disposable but still capable of effectuating a substantially aseptic seal to a vessel while still allowing maximum flexibility in inserts. In addition, the methods of utilizing the vessel closures to circulate fluid into and out of a vessel provide systems with relatively few components all while maintaining a substantially aseptic system in which the fluid is located.

The vessel closures may be assembled and then the entire devices or components thereof may be rendered substantially aseptic by, for example, gamma radiation. Alternatively, the entire vessel closures or components thereof may be rendered substantially aseptic by exposure to steam above 121°C for a period of time long enough to eliminate microorganisms. The entire devices or components thereof may also be rendered aseptic by chemical treatment, such as with ethylene oxide (ETO). Once rendered substantially aseptic, the vessel closures may be appropriately packaged and stored to maintain the substantially aseptic state until ready for use.

The foregoing descriptions of vessel closures, methods of manufacturing vessel closures, and methods of utilizing vessel closures illustrate and describe various embodiments. As various changes can be made in the above embodiments without departing from the scope of the invention disclosed and claimed herein, it is intended that all matter contained in the above description or shown in the accompanying figures shall be interpreted as illustrative and not limiting. Furthermore, the scope of the invention covers various modifications, combinations, alterations, etc., of the above-described embodiments that all are within the scope of the claims. Additionally, the disclosure shows and describes only selected embodiments of the invention, but the invention is capable of use in various other combinations, modifications, and environments and is capable of changes or modifications within the scope of the claims, commensurate with the above teachings, and/or within the skill or knowledge of artisans in the relevant art. Furthermore, certain features and characteristics of each embodiment may be selectively interchanged and applied to other illustrated and non-illustrated embodiments of the invention without departing from the scope of the invention as defined by the claims.

## Claims

1. A fluid transfer hub (6000), comprising:
a first vessel closure (6010);
a second vessel closure (6015),
a gasket (6020) arranged between the first vessel closure (6010) and the second vessel closure (6015); and
a clamp (6005) at least partially surrounding the first vessel closure (6010), the second vessel closure (6015) and the gasket (6020);
wherein the first vessel closure (6010) comprises:
a first body (6025), one or more apertures extending axially through the first body (6025), one or more first inserts (6035) extending axially through the one or more apertures, and a cast seal (6030) disposed within the first body (6025) and surrounding each first insert (6035),
wherein the second vessel closure (6015) comprises:
a second body (6025), one or more apertures extending axially through the second body, one or more second inserts (6035) extending axially through the one or more apertures, and a cast seal (6030) disposed within the second body and surrounding each second insert (6035).

2. The fluid transfer hub of claim 1, wherein a chamber (6050) is defined between the first vessel closure and the second vessel closure.

3. The fluid transfer hub of claim 2, wherein the gasket (6020) comprises one of a screen gasket, a perforated plate gasket, and an orifice gasket, wherein the gasket bisects the chamber

4. The fluid transfer hub of claim 2 or claim 3, wherein each surface of each body (6025) that defines the chamber (6050) is covered by the cast seal, (6030) preferably wherein the cast seal contacts the gasket.

5. The fluid transfer hub of any one of claims 2-4, wherein the cast seal (6030) comprises a curved edge between the chamber (6050) and each insert (6035).

6. The fluid transfer hub of any one of claims 1-5, wherein the quantity of first inserts equals one and the quantity of second inserts is greater than one such that fluid entering through the first vessel closure is distributed by the second vessel closure.

7. The fluid transfer hub of any one of claims 1-6, wherein the cast seal (6030) is formed by a casting agent, and the casting agent is liquid silicone and/or perfluoropolyether.

8. The fluid transfer hub of any one of claims 1-7, wherein the cast seal (6030) is attached to the body (6025) by way of priming at least a portion of the body (6025) and adhesively attaching the cast seal (6030) to the body, and/or wherein the cast seal (6030) is aseptically secured to the first insert (6035) and second insert (6035) respectively.

9. The fluid transfer hub of any one of claims 1-8, wherein at least one of the first inserts and the second inserts comprise silicone tubes.

10. The fluid transfer hub of claim 9, wherein the silicone tubes are adhesively attached to one or more anchors, (6055), preferably wherein the one or more anchors are constructed from material selected from the group consisting of metal, plastic, glass-filled plastic, ceramics, and composites.

11. The fluid transfer hub of any one of claims 1-10, wherein at least one of the first inser (6035) and the second insert (6035) comprises tubing; and
wherein a deformable sleeve is adhesively attached to and surrounding at least a portion of the tubing;
wherein the sleeve is formed of a material having plasticity such that pressure applied to the sleeve causes the sleeve to deform about and seal the tubing and upon continued application of pressure to the sleeve, the sleeve and tubing are cut and the sleeve retains a deformed shape substantially sealing the tubing.

12. The fluid transfer hub of any one of claims 1-11, further comprising a primer applied to one of the first body (6025), the second body (6025), the first insert (6035) or the second insert (6035).

13. A method of manufacturing a fluid transfer hub, the method comprising:
creating a first vessel closure (6010);
creating a second vessel closure (6015);
positioning a gasket (6020) between the first vessel closure and the second vessel closure; and
securing the first vessel closure to the second vessel closure with a clamp (6005);
wherein creating the first vessel closure and creating the second vessel closure each comprises:
inserting at least one tube into at least one aperture in a body (6025);
casting a casting agent into the body (6025) after inserting the at least one tube; and
curing the casting agent to form a seal around and bond the at least one tube to the body (6025).

14. The method ofclaim 13, wherein creating the first vessel closure comprises inserting a single tube into a single aperture, and wherein creating the second vessel closure comprises inserting a plurality of tubes into a plurality of apertures.

15. The method of claim 13 or claim 14, wherein creating at least the first vessel closure comprises:
obtaining a body (6025) having one or more apertures extending axially through the body: extending one or more inserts axially through the one or more apertures;
engaging a cast form (6070) with the body, the cast form comprising at least one boss (6080) extending from a base surface thereof, wherein a curved surface transitions from the base surface to the boss; and
casting a casting agent in a cavity formed between the body (6025) and the cast form (6070).

## Patentansprüche

1. Fluidtransfernabe (6000), die Folgendes umfasst:
einen ersten Behälterverschluss (6010);
einen zweiten Behälterverschluss (6015),
einen Dichtring (6020), der sich zwischen dem ersten Behälterverschluss (6010) und dem zweiten Behälterverschluss (6015) befindet; und
eine Klemme (6005), die den ersten Behälterverschluss (6010), den zweiten Behälterverschluss (6015) und die Dichtung (6020) mindestens teilweise umgibt;
wobei der erste Behälterverschluss (6010) Folgendes umfasst:
einen ersten Körper (6025), eine oder mehrere Öffnungen, die sich axial durch den ersten Körper (6025) erstrecken, einen oder mehrere erste Einsätze (6035), die sich axial durch die eine oder die mehreren Öffnungen erstrecken, und eine Gussdichtung (6030), die im ersten Körper (6025) angeordnet ist und jeden ersten Einsatz (6035) umgibt,
wobei der zweite Behälterverschluss (6015) Folgendes umfasst:
einen zweiten Körper (6025), eine oder mehrere Öffnungen, die sich axial durch den zweiten Körper erstrecken, einen oder mehrere zweite Einsätze (6035), die sich axial durch die eine oder die mehreren Öffnungen erstrecken, und eine Gussdichtung (6030), die im zweiten Körper angeordnet ist und jeden zweiten Einsatz (6035) umgibt.

2. Fluidtransfernabe nach Anspruch 1, wobei zwischen dem ersten Behälterverschluss und dem zweiten Behälterverschluss eine Kammer (6050) definiert ist.

3. Fluidtransfernabe nach Anspruch 2, wobei der Dichtring (6020) eines von einem Siebdichtring, einem perforierten Plattendichtring und einen Blendendichtring umfasst, wobei der Dichtring die Kammer halbiert.

4. Fluidtransfernabe nach Anspruch 2 oder Anspruch 3, wobei jede Fläche jedes Körpers (6025), der die Kammer (6050) definiert, durch die Gussdichtung (6030) abgedeckt wird, vorzugsweise wobei die Gussdichtung den Dichtring berührt.

5. Fluidtransfernabe nach einem der Ansprüche 2-4, wobei die Gussdichtung (6030) zwischen der Kammer (6050) und jedem Einsatz (6035) einen gekrümmten Rand umfasst.

6. Fluidtransfernabe nach einem der Ansprüche 1-5, wobei die Menge von ersten Einsätzen gleich eins ist und die Menge von zweiten Einsätzen größer als eins ist, derart, dass Fluid, das durch den ersten Behälterverschluss eintritt, vom zweiten Behälterverschluss verteilt wird.

7. Fluidtransfernabe nach einem der Ansprüche 1-6, wobei die Gussdichtung (6030) durch ein Gussmittel gebildet wird und das Gussmittel flüssiges Silikon und/oder Perfluorpolyether ist.

8. Fluidtransfernabe nach einem der Ansprüche 1-7, wobei die Gussdichtung (6030) durch Grundieren von mindestens einem Abschnitt des Körpers (6025) und klebendes Befestigen der Gussdichtung (6030) am Körper am Körper (6025) befestigt wird und/oder wobei die Gussdichtung (6030) am ersten Einsatz (6035) bzw. am zweiten Einsatz (6035) aseptisch gesichert wird.

9. Fluidtransfernabe nach einem der Ansprüche 1-8, wobei mindestens einer der ersten Einsätze und der zweiten Einsätze Silikonschläuche umfasst.

10. Fluidtransfernabe nach Anspruch 9, wobei die Silikonschläuche klebend an einem oder mehreren Ankern (6055) befestigt sind, vorzugsweise wobei der eine oder die mehreren Anker aus einem Material konstruiert sind, das ausgewählt ist aus der Gruppe, die aus Metall, Kunststoff, glasgefülltem Kunststoff, Keramik und Verbundstoffen besteht.

11. Fluidtransfernabe nach einem der Ansprüche 1-10, wobei mindestens einer des ersten Einsatzes (6035) und des zweiten Einsatzes (6035) einen Schlauch umfasst und
wobei eine verformbare Hülse an mindestens einem Abschnitt des Schlauches klebend befestigt ist und denselben umgibt;
wobei die Hülse aus einem Material gebildet ist, das eine Plastizität aufweist, derart, dass Druck, der auf die Hülse ausgeübt wird, bewirkt, dass sich die Hülse um den Schlauch verformt und denselben abdichtet, und bei fortgesetzter Ausübung von Druck auf die Hülse die Hülse und der Schlauch abgeschnitten werden und die Hülse eine verformte Form beibehält, wodurch der Schlauch im Wesentlichen abgedichtet wird.

12. Fluidtransfernabe nach einem der Ansprüche 1-11, die ferner eine Grundierung umfasst, die auf einen des ersten Körpers (6025), des zweiten Körpers (6025), des ersten Einsatzes (6035) oder des zweiten Einsatzes (6035) aufgebracht wird.

13. Verfahren zum Herstellen einer Fluidtransfernabe, wobei das Verfahren Folgendes umfasst:
Erstellen eines ersten Behälterverschlusses (6010);
Erstellen eines zweiten Behälterverschlusses (6015);
Positionieren eines Dichtrings (6020) zwischen dem ersten Behälterverschluss und dem zweiten Behälterverschluss und
Sichern des ersten Behälterverschlusses mit einer Klemme (6005) am zweiten Behälterverschluss;
wobei das Erstellen des ersten Behälterverschlusses und das Erstellen des zweiten Behälterverschlusses jeweils Folgendes umfasst:
Einstecken von mindestens einem Schlauch an mindestens einer Öffnung in einen Körper (6025);
nach Einstecken des mindestens einen Schlauches Gießen eines Gussmittels in den Körper (6025) und
Aushärten des Gussmittels, um eine Dichtung um den mindestens einen Schlauch zu bilden und denselben an den Körper (6025) zu bonden.

14. Verfahren nach Anspruch 13, wobei das Erstellen des ersten Behälterverschlusses das Einstecken eines einzelnen Schlauches in eine einzelne Öffnung umfasst und wobei das Erstellen des zweiten Behälterverschlusses das Einstecken einer Vielzahl von Schläuchen in eine Vielzahl von Öffnungen umfasst.

15. Verfahren nach Anspruch 13 oder Anspruch 14, wobei das Erstellen mindestens des ersten Behälterverschlusses Folgendes umfasst:
Erhalten eines Körpers (6025), der eine oder mehrere Öffnungen aufweist, die sich axial durch den Körper erstrecken;
Erstrecken von einem oder mehreren Einsätzen axial durch die eine oder die mehreren Öffnungen;
Einrücken einer Gussform (6070) in den Körper, wobei die Gussform mindestens eine Erhöhung (6080) umfasst, die sich von einer Basisfläche davon erstreckt, wobei eine gekrümmte Fläche von der Basisfläche zur Erhöhung übergeht; und
Gießen eines Gussmittels in einen Hohlraum, der zwischen dem Körper (6025) und der Gussform (6070) gebildet ist.

## Revendications

1. Raccord de transfert de fluide (6000) comprenant :
une première fermeture de récipient (6010) ;
une seconde fermeture de récipient (6015),
un joint (6020) agencé entre la première fermeture de récipient (6010) et la seconde fermeture de récipient (6015) ; et
un clamp (6005) entourant au moins partiellement la première fermeture de récipient (6010), la seconde fermeture de récipient (6015) et le joint (6020) ;
dans lequel la première fermeture de récipient (6010) comprend :
un premier corps (6025), une ou plusieurs ouvertures s'étendant axialement à travers le premier corps (6025), un ou plusieurs premiers inserts (6035) s'étendant axialement à travers les une ou plusieurs ouvertures, et un joint d'étanchéité moulé (6030) disposé dans le premier corps (6025) et entourant chaque premier insert (6035),
dans lequel la seconde fermeture de récipient (6015) comprend :
un second corps (6025), une ou plusieurs ouvertures s'étendant axialement à travers le second corps, un ou plusieurs seconds inserts (6035) s'étendant axialement à travers les une ou plusieurs ouvertures, et un joint d'étanchéité moulé (6030) disposé dans le second corps, et entourant chaque second insert (6035).

2. Raccord de transfert de fluide selon la revendication 1, dans lequel une chambre (6050) est définie entre la première fermeture de récipient et la seconde fermeture de récipient.

3. Raccord de transfert de fluide selon la revendication 2, dans lequel le joint (6020) comprend un joint parmi un joint d'écran, un joint de plaque perforé et un joint d'orifice, dans lequel le joint coupe la chambre.

4. Raccord de transfert de fluide selon la revendication 2 ou la revendication 3, dans lequel chaque surface de chaque corps (6025) qui définit la chambre (6050) est recouverte par le joint d'étanchéité moulé (6030), de préférence dans lequel le joint d'étanchéité moulé est en contact avec le joint.

5. Raccord de transfert de fluide selon l'une quelconque des revendications 2 à 4, dans lequel le joint d'étanchéité moulé (6030) comprend un bord incurvé entre la chambre (6050) et chaque insert (6035).

6. Raccord de transfert de fluide selon l'une quelconque des revendications 1 à 5, dans lequel la quantité de premiers inserts est égale à un et la quantité de seconds inserts est supérieure à un, de sorte que le fluide entrant par la première fermeture de récipient est distribué par la seconde fermeture de récipient.

7. Raccord de transfert de fluide selon l'une quelconque des revendications 1 à 6, dans lequel le joint d'étanchéité moulé (6030) est formé par un agent de moulage, et l'agent de moulage est de la silicone liquide et/ou du perfluoropolyéther.

8. Raccord de transfert de fluide selon l'une quelconque des revendications 1 à 7, dans lequel le joint d'étanchéité moulé (6030) est fixé au corps (6025) au moyen de l'amorçage d'au moins une partie du corps (6025) et en fixant par voie adhésive le joint d'étanchéité moulé (6030) au corps, et/ou dans lequel le joint d'étanchéité moulé (6030) est fixé, de manière aseptique, au premier insert (6035) et au second insert (6035), respectivement.

9. Raccord de transfert de fluide selon l'une quelconque des revendications 1 à 8, dans lequel au moins l'un parmi les premiers inserts et les seconds inserts comprend des tubes de silicone.

10. Raccord de transfert de fluide selon la revendication 9, dans lequel les tubes de silicone sont fixés, de manière adhésive, à un ou plusieurs ancrages (6055),
de préférence, dans lequel les un ou plusieurs ancrages sont construits à partir d'un matériau sélectionné dans le groupe comprend le métal, le plastique, le plastique renforcé en fibres de verre, la céramique et les composites.

11. Raccord de transfert de fluide selon l'une quelconque des revendications 1 à 10, dans lequel au moins l'un parmi le premier insert (6035) et le second insert (6035) comprend un tubage ; et
dans lequel un manchon déformable est fixé, par voie adhésive à et entourant au moins une partie du tubage ;
dans lequel le manchon est formé avec un matériau présentant une plasticité de sorte que la pression appliquée sur le manchon amène le manchon à se déformer autour et à sceller le tubage et suite à l'application continue de pression sur le manchon, le manchon et le tubage sont coupés et le manchon conserve une forme déformée scellant sensiblement le tubage.

12. Raccord de transfert de fluide selon l'une quelconque des revendications 1 à 11, comprenant en outre une amorce appliquée sur l'un parmi le premier corps (6025), le second corps (6025), le premier insert (6035) ou le second insert (6035) .

13. Procédé pour fabriquer un raccord de transfert de fluide, le procédé comprenant les étapes suivantes:
créer une première fermeture de récipient (6010);
créer une seconde fermeture de récipient (6015);
positionner un joint (6020) entre la première fermeture de récipient et la seconde fermeture de récipient ; et
fixer la première fermeture de récipient à la seconde fermeture de récipient avec un clamp (6005) ;
dans lequel l'étape pour créer la première fermeture de récipient et l'étape pour créer la seconde fermeture de récipient comprennent chacune les étapes suivantes :
insérer au moins un tube dans au moins une ouverture dans un corps (6025) ;
mouler un agent de moulage dans le corps (6025) après avoir inséré le au moins un tube ; et
faire durcir l'agent de moulage afin de former un joint d'étanchéité autour et relier le au moins un tube au corps (6025).

14. Procédé selon la revendication 13, dans lequel l'étape pour créer la première fermeture de récipient comprend l'étape pour insérer un seul tube dans une seule ouverture, et dans lequel l'étape pour créer la seconde fermeture de récipient comprend l'étape pour insérer une pluralité de tubes dans une pluralité d'ouvertures.

15. Procédé selon la revendication 13 ou la revendication 14, dans lequel l'étape pour créer au moins la première fermeture de récipient comprend les étapes suivantes :
obtenir un corps (6025) ayant une ou plusieurs ouvertures s'étendant axialement à travers le corps ;
étendre un ou plusieurs inserts axialement à travers les une ou plusieurs ouvertures ;
mettre en prise une forme moulée (6070) avec le corps, la forme moulée comprenant au moins un bossage (6080) s'étendant à partir de sa surface de base, dans lequel une surface incurvée effectue une transition de la surface de base au bossage ; et
mouler un agent de moulage dans une cavité formée entre le corps (6025) et la forme moulée (6070).
